# EUROPÄISCHE PATENTANMELDUNG

(11) **EP 3 985 389 A1**
(43) Veröffentlichungstag der Anmeldung: **20.04.2022**
(21) Anmeldenummer: 20202209.1
(22) Anmeldetag: 16.10.2020
(51) Int. Cl.: G01N 30/46, G01N 30/88, G01N 30/78, G01N 30/86

(54) **GASCHROMATOGRAPH ZUR ANALYSE VON ERDGAS ODER ÄHNLICHEN GASGEMISCHEN**

(71) Anmelder: Siemens Aktiengesellschaft, 80333 München (DE)
(72) Erfinder: Strauch, Piotr, 76761 Rülzheim (DE)

(57) **Zusammenfassung**

Gaschromatograph zur Analyse von Erdgas oder ähnlichen Gasgemischen (1) mit in Reihe geschalteten Trennsäulen (13, 15, 17, 19) und diesen nachgeordneten Detektoren (14, 16, 18, 20), wobei die erste Trennsäule (13) und der erste Detektor (14) zur Trennung bzw. Detektion von n-Hexan und höheren Kohlenwasserstoffen, die zweite Trennsäule (15) und der zweite Detektor (16) zur Trennung bzw. Detektion der Isomeren von Butan und Pentan und die dritte Trennsäule (17) und der dritte Detektor (18) zur Trennung bzw. Detektion von Kohlendioxid, Ethan, Schwefelwasserstoff, Wasser und Propan ausgebildet sind.

Mit Hilfe einer vierten Trennsäule (19) und eines vierten Detektors (20) können Wasserstoff, Sauerstoff, Stickstoff, Methan ggf. auch Helium und Kohlenmonoxid detektiert werden, wobei zwischen dem dritten Detektor (18) und der vierten Trennsäule (19) eine steuerbare Umschalteinrichtung (28) zur Ausschleusung von dem aus der dritten Trennsäule (17) eluierenden Kohlendioxid und folgenden Eluaten vorgesehen ist.

Das Wasser und der Schwefelwasserstoff werden unter Verwendung von relativen Responsefaktoren des dritten Detektors (18) für Wasser und Schwefelwasserstoff zu Ethan, Kohlendioxid oder Propan quantitativ bestimmt.

## Beschreibung

Die Norm DIN EN ISO 6976:2016 legt Verfahren für die Berechnung brenntechnisch relevanter Größen wie Brennwert, Heizwert, Dichte, relativer Dichte sowie oberem und unterem Wobbe-Index von Erdgasen, Erdgasaustauschgasen und sonstigen Brenngasen fest, wenn die Zusammensetzung der Gasgemische in Stoffmengenanteilen bekannt ist. Anhand der festgelegten Verfahren können die Eigenschaften des Gasgemisches bei allgemein üblichen Referenzbedingungen berechnet werden.

Die Messung der Zusammensetzung von Erdgas erfolgt üblicherweise mit Hilfe eines Gaschromatographen, in dem die Komponenten des Gasgemischs chromatographisch getrennt und nacheinander detektiert werden. Anhand der quantitativ bestimmten Komponenten können dann die interessierenden brenntechnisch relevanten Größen berechnet werden.

Durch einzügige (single-train) chromatographische Analyse mit hintereinander geschalteten Trennsäulen kann die Zusammensetzung von Erdgas besonders schnell mit kurzen Zykluszeiten von nur wenigen Minuten ermittelt werden.

Die Erfindung betrifft einen entsprechenden Gaschromatographen zur Analyse von Erdgas oder ähnlichen Gasgemischen
- mit einer Dosiereinrichtung, die eine Probe des Gasgemischs in einen Trägergasstrom einschleust,
- mit einer von dem Trägergasstrom und der eingeschleusten Probe durchströmten und Gaskomponenten des Gasgemischs trennenden Trenneinrichtung, in der in Strömungsrichtung eine erste Trennsäule gefolgt von einem ersten Detektor, eine zweite Trennsäule gefolgt von einem zweiten Detektor und eine dritte Trennsäule gefolgt von einem dritten Detektor in einer Reihenschaltung angeordnet sind, wobei die Detektoren in Abhängigkeit von ankommenden getrennten Gaskomponenten Detektorsignale erzeugen, und
- mit einer an den Detektoren angeschlossenen Auswerteeinrichtung, die in Abhängigkeit von den Detektorsignalen zumindest einen Teil der getrennten Gaskomponenten quantitativ bestimmt.

Bei einem derartigen aus der EP 1 488 226 B1 bekannten Gaschromatographen sind die erste Trennsäule und der erste Detektor zur Trennung bzw. Detektion von Propan und höheren Kohlenwasserstoffen bis n-Hexan ausgebildet, die zweite Trennsäule und der zweite Detektor zur Trennung bzw. Detektion von Kohlendioxid und Ethan ausgebildet und die dritte Trennsäule und der dritte Detektor zur Trennung bzw. Detektion von Stickstoff und Methan und erforderlichenfalls Sauerstoff und Kohlenmonoxid ausgebildet. Zwischen der zweiten Trennsäule und der dritten Trennsäule ist eine steuerbare Umschalteinrichtung vorhanden, mittels derer auf das Methan folgende Eluate aus der Trenneinrichtung ausgeschleust werden, um sie von der dritten Trennsäule fernzuhalten. Um höhere Kohlenwasserstoffe als n-Hexan detektieren zu können, werden nach der Detektion des n-Hexan durch den ersten Detektor die erste und zweite Trennsäule über die Umschalteinrichtung mit Trägergas rückgespült, wobei diese höheren Kohlenwasserstoffe mittels eines vor der ersten Trennsäule angeordneten Detektors ungetrennt und in Summe detektiert werden. Die steuerbare Umschalteinrichtung arbeitet ohne Ventile (sog. Live-Schaltung), so wie auch ein ventilloser Injektor vorgesehen ist, um die in den Trägergasstrom eingeschleuste Probe nochmals scharf zu begrenzen.

Aus der WO 2016/096423 A1 ist ein Gaschromatograph bekannt, bei dem die Probendosierung und Trennsäulenumschaltung mittels einer Mehrwegeventileinheit erfolgt. In dem vor der Trennsäulenumschaltung liegenden vorderen Teil der Trenneinrichtung werden Hochsieder wie Propan, Butan und höhere Kohlenwasserstoffe getrennt und detektiert, während in dem hinter der Trennsäulenumschaltung liegenden Teil der Trenneinrichtung Niedrigsieder wie Stickstoff, Kohlenmonoxid, Kohlendioxid, Ethan und Schwefelwasserstoff getrennt und detektiert werden. Hexan und höhere Kohlenwasserstoffe werden in Summe in einem Detektor vor dem vorderen Teil der Trenneinrichtung detektiert, indem diese mit Trägergas rückgespült wird.

Für eine genaue Bestimmung von brenntechnisch relevanten Größen von Erdgas oder ähnlichen Gasgemischen ist die Ermittlung des Anteils von Wasser in dem betreffenden Gasgemisch wichtig. Ferner ist es wünschenswert, höhere Kohlenwasserstoffe als n-Hexan differenziert und nicht wie bisher lediglich in Summe zu ermitteln.

Der Erfindung liegt daher die Aufgabe zugrunde, eine umfassende chromatographische Analyse von Erdgas oder ähnlichen Gasgemischen zu ermöglichen.

Gemäß der Erfindung wird die Aufgabe durch den in Anspruch 1 angegebenen Gaschromatographen gelöst, von dem vorteilhafte Weiterbildungen in den Unteransprüchen angegeben sind.

Gegenstand der Erfindung ist somit ein Gaschromatograph zur Analyse von Erdgas oder ähnlichen Gasgemischen
- mit einer Dosiereinrichtung, die eine Probe des Gasgemischs in einen Trägergasstrom einschleust,
- mit einer von dem Trägergasstrom und der eingeschleusten Probe durchströmten und Gaskomponenten des Gasgemischs trennenden Trenneinrichtung, in der in Strömungsrichtung eine erste Trennsäule gefolgt von einem ersten Detektor, eine zweite Trennsäule gefolgt von einem zweiten Detektor und eine dritte Trennsäule gefolgt von einem dritten Detektor in einer Reihenschaltung angeordnet sind, wobei die Detektoren in Abhängigkeit von ankommenden getrennten Gaskomponenten Detektorsignale erzeugen, und
- mit einer an den Detektoren angeschlossenen Auswerteeinrichtung, die in Abhängigkeit von den Detektorsignalen zumindest einen Teil der getrennten Gaskomponenten quantitativ bestimmt, wobei
- die erste Trennsäule und der erste Detektor zur Trennung bzw. Detektion von n-Hexan und höheren Kohlenwasserstoffen ausgebildet sind,
- die zweite Trennsäule und der zweite Detektor zur Trennung bzw. Detektion der Isomeren von Butan und Pentan ausgebildet sind,
- die dritte Trennsäule und der dritte Detektor zur Trennung bzw. Detektion von Kohlendioxid, Ethan, Schwefelwasserstoff, Wasser und Propan ausgebildet sind und wobei
- die Auswerteeinrichtung ferner dazu ausgebildet ist, das Wasser und den Schwefelwasserstoff anhand der von dem dritten Detektor für das Wasser bzw. den Schwefelwasserstoff gelieferten Detektorsignale und in einem Kalibrierspeicher der Auswerteeinrichtung gespeicherten relativen Responsefaktoren des dritten Detektors für Wasser und Schwefelwasserstoff zu jeweils einer Bezugskomponente, insbesondere zu Ethan, Kohlendioxid oder Propan, quantitativ zu bestimmen.

Die Trennung von Schwefelwasserstoff, Wasser und Propan in der dritten Trennsäule ermöglicht zum einen die Detektion und Bestimmung des Wasseranteils in dem Gasgemisch und verringert zum anderen den verfälschenden Einfluss von Wasser auf die Messung von Propan, wie dies der Fall sein kann, wenn, wie bisher, Propan zusammen mit Butan und höheren Kohlenwasserstoffe getrennt und detektiert wird.

Grundsätzlich kann für jede zu bestimmende Gaskomponente eine eigene Kalibrierung erfolgen, indem dem Gaschromatographen eine Kalibrierprobe aufgegeben wird, die die jeweilige Komponente in einer bekannten Konzentration cᵢ enthält. Damit lässt sich für den die betreffende Gaskomponente detektierenden Detektor ein absoluter Responsefaktor RFᵢ = A_{i,cal}/c_{i,cal} ermitteln, wobei c_{i,cal} die Konzentration der Komponente i in der Kalibrierprobe und A_{i,cal} die resultierende Detektorantwort in Form einer Peakfläche unter dem Detektorsignal bezeichnen. Dies kann sehr aufwendig sein, und in vielen Fällen stehen auch keine Kalibriergasgemische zur Verfügung, die alle zu messenden Gaskomponenten enthalten. So enthalten typisehe Kalibriergasgemische keine Komponenten wie Wasser, Schwefelwasserstoff, außer n-Hexan keine weiteren Hexan-Isomeren und auch keine höhere Kohlenwasserstoffe. Die Herstellung, der Transport, die Lagerung und Handhabung von diese Komponenten bzw. Subkomponenten enthaltenden komplexen Kalibriergasgemischen wäre sehr schwierig und mit vielen möglichen Fehlerquellen aufgrund des komplexeren Herstellungsprozesses oder z. B. der Kondensation von Wasser während der Lagerung bzw. des Transports verbunden. Die Verwendung solcher Kalibriergasgemische würde die Fehleranfälligkeit und die Betriebskosten erhöhen. Außerdem ist es schwierig, bei der Probenaufgabe jedes Mal dieselbe Probenmenge mit hoher Genauigkeit reproduzierbar zu dosieren. Dies gilt besonders für die Komponenten Wasser und Schwefelwasserstoff.

Die Konzentrationen cᵢ von Komponenten i, für die kein eigener Responsefaktor RFᵢ vorliegt bzw. durch Kalibration ermittelt wurde, können jedoch mit Hilfe von relativen Responsefaktoren RRFᵢ₋ₖ und dem Responsefaktor RFₖ einer anderen Gaskomponente (Bezugskomponente) k wie folgt bestimmt werden: cᵢ = Aᵢ/(RFₖ·RRFᵢ₋ₖ). Die relativen Responsefaktoren RRFᵢ₋ₖ haben einen universellen Charakter und können unter kontrollierten bzw. Laborbedienungen und unabhängig von der Anwendung ermittelt werden. Bei der Ermittlung von relativen Responsefaktoren können dem Kalibriergasgemisch mit der Bezugskomponente k in bekannter Konzentration cₖ weitere Gaskomponenten i mit bekannter Konzentration cᵢ hinzugefügt werden, wobei der der relative Responsefaktor RRFᵢ₋ₖ der Komponente i zur Komponente k wie folgt ermittelt werden kann: RRFᵢ₋ₖ = (A_{i,cal}/C_{i,cal}) / (A_{k,cal}/c_{kcal}).

Bei dem erfindungsgemäßen Gaschromatograph wird nun der Anteil des Wassers in dem zu analysierenden Gasgemisch anhand des von dem dritten Detektor gelieferten Detektorsignals und eines in einem Kalibrierspeicher der Auswerteeinrichtung gespeicherten relativen Responsefaktors des dritten Detektors für Wasser zu einer Bezugskomponente bestimmt, bei der es sich um eine der ebenfalls von dem dritten Detektor detektierten und in der Auswerteeinrichtung quantitativ bestimmten Gaskomponenten Ethan, Kohlendioxid oder Propan handelt. Bei dem relativen Responsefaktor RRFᵢ₋ₖ bezeichnet dann der Index i Wasser und der Index k z. B. Ethan, das besonders geeignet ist, weil es normalerweise in höheren Konzentrationen vorliegt als die anderen genannten Gaskomponenten.

Auf die gleiche Weise wird auch der Anteil von Schwefelwasserstoff in dem zu analysierenden Gasgemisch unter Verwendung eines relativen Responsefaktors des dritten Detektors für Schwefelwasserstoff zu einer der Bezugskomponenten Ethan, Kohlendioxid oder Propan ermittelt.

Alternativ könnten relative Responsefaktoren zu anderen Gaskomponenten verwendet werden, die von anderen Detektoren detektiert werden. Dazu müsste aber ein Proportionalitäts- oder Skalierfaktor zwischen den Detektoren bekannt sein bzw. ermittelt werden.

Die relativen Responsefaktoren vom Wasser und Schwefelwasserstoff zu den anderen einfach bestimmbaren Messkomponenten wie Ethan Propan oder Kohlendioxid werden vorzugsweise unter Laborbedienungen ermittelt. Wenigstens ein Kalibriergasgemisch mit bekanntem Stoffmengenanteil von Wasser bzw. Schwefelwasserstoff und der Bezugskomponente wird der Analyse zugeführt und zur Ermittlung des jeweiligen relativen Responsefaktors verwendet. Wenn z. B. der genaue Wassergehalt in dem Kalibriergemisch unbekannt ist, kann er mittels weiterer analytische Methoden, wie z. B. Spektrometrie, bestimmt werden.

Alternativ zur Bestimmung der relativen Responsefaktoren im Labor können sie auch bei der Inbetriebnahme im Feld oder bei einer periodisch, z. B. jährlich, stattfindenden Inspektion mit Hilfe von komplexeren Kalibriergemischen unter Einhaltung besonderer Sorgfalt hinsichtlich Flaschenhandhabung, Probenzufuhr und Stabilität der Messung ermittelt werden.

Die ermittelten relativen Responsefaktoren werden in einem Kalibrierspeicher der Auswerteeinrichtung gespeichert und zur Bestimmung der Konzentrationen (Stoffmengenanteile) von Wasser und Schwefelwasserstoff im Messbetrieb verwendet.

Während des Regelbetriebs des Gaschromatographen können dann Kalibrationen mit Hilfe von einfachen Kalibriergasgemischen durchgeführt werden, die frei von Wasser und Schwefelwasserstoff sind. Kalibriert werden dann unter anderem auch die jeweils für Wasser bzw. Schwefelwasserstoff verwendeten Bezugskomponenten.

Die erste Trennsäule dient zur Trennung von n-Hexan und höheren Kohlenwasserstoffen wie Benzol, Heptan, Methylcyclohexan, Toluol und erforderlichenfalls Octan und Nonan, die einzeln detektiert werden. Dabei können die höheren Kohlenwasserstoffe als n-Hexan anhand der von dem ersten Detektor für sie gelieferten Detektorsignale und von relativen Responsefaktoren des ersten Detektors für diese höheren Kohlenwasserstoffe in Bezug auf n-Hexan quantitativ bestimmen werden.

Die erste Trennsäule und der erste Detektor können in vorteilhafter Weise weiterhin zur Trennung bzw. Detektion der von n-Hexan verschiedenen Hexan-Isomeren 2-Methylpentan (Isohexan), 3-Methylpentan, 2,2-Dimethylbutan (Neohexan), 2,3-Dimethylbutan ausgebildet sein, wobei diese Hexan-Isomeren in Abhängigkeit von dem Detektorsignal des ersten Detektors und relativen Responsefaktoren des ersten Detektors für diese Hexan-Isomeren gegenüber n-Hexan quantitativ zu bestimmen.

Alternativ können die von n-Hexan verschiedenen Hexan-Isomeren auch in der zweiten Trennsäule getrennt und durch den zweiten Detektor detektiert werden. Die quantitative Bestimmung erfolgt dann in Abhängigkeit von den Detektorsignal des zweiten Detektors und relativen Responsefaktoren des zweiten Detektors für diese Hexan-Isomeren gegenüber n-Pentan oder alternativ gegenüber n-Hexan, das zu diesem Zweck ebenfalls von dem zweiten Detektor detektiert wird, d. h. der zweite Detektor detektiert in diesem Fall auch die Hexan-Isomeren.

Die verwendeten relativen Responsefaktoren werden kontrollierten Bedingungen für die betreffenden Detektoren ermittelt und in dem Kalibrierspeicher der Auswerteeinrichtung gespeichert.

Die dritte Trennsäule und der dritte Detektor können in vorteilhafter Weise dazu ausgebildet sein, neben dem Kohlendioxid, Ethan, Schwefelwasserstoff, Wasser und Propan auch noch Stickstoff, Methan und erforderlichenfalls auch Wasserstoff zu trennen und zu detektieren. Dabei ist zwischen dem zweiten Detektor und der dritten Trennsäule eine steuerbare Umschalteinrichtung zur Ausschleusung von auf das Propan folgenden Eluaten aus der zweiten Trennsäule vorgesehen. Dadurch kann die Analysenzykluszeit kurz gehalten werden, weil ansonsten Butan und die weiteren höheren Kohlenwasserstoffe in der dritten Trennsäule vergleichsweise lang eluieren würden und bei nicht ausreichend langer Spülzeit die nachfolgenden Messungen störe würden.

Um insbesondere auch den Anteil von Sauerstoff und Wasserstoff in dem Gasgemisch bestimmen zu können, kann die Trennung und Detektion von Stickstoff und Methan nicht durch die dritte Trennsäule und den dritten Detektor sondern in einer nachgeordneten vierten Trennsäule mit viertem Detektor durchgeführt werden, die zur Trennung bzw. Detektion von Wasserstoff, Sauerstoff, Stickstoff, Methan ggf. auch Helium und Kohlenmonoxid ausgebildet sind. Die steuerbare Umschalteinrichtung ist dann zwischen dem dritten Detektor und der vierten Trennsäule vorgesehen und dient zur Ausschleusung des aus der dritten Trennsäule eluierenden Kohlendioxids und den folgenden Eluaten.

Im Weiteren wird die Erfindung anhand von Ausführungsbeispielen und unter Bezugnahme auf die Figuren der Zeichnung erläutert; im Einzelnen zeigen
- Fig. 1 bis 4: ein Ausführungsbeispiel des erfindungsgemäßen Gaschromatographen mit vier Trennsäulen und einer aus zehn Ventilen bestehenden Mehrwegeventileinheit in vier unterschiedlichen Schaltstellungen,
- Fig. 5: ein weiteres Ausführungsbeispiel des erfindungsgemäßen Gaschromatographen mit drei Trennsäulen.

Gleiche Bezugszeichen haben in den verschiedenen Figuren die gleiche Bedeutung. Die Darstellungen sind rein schematisch und repräsentieren keine Größenverhältnisse.

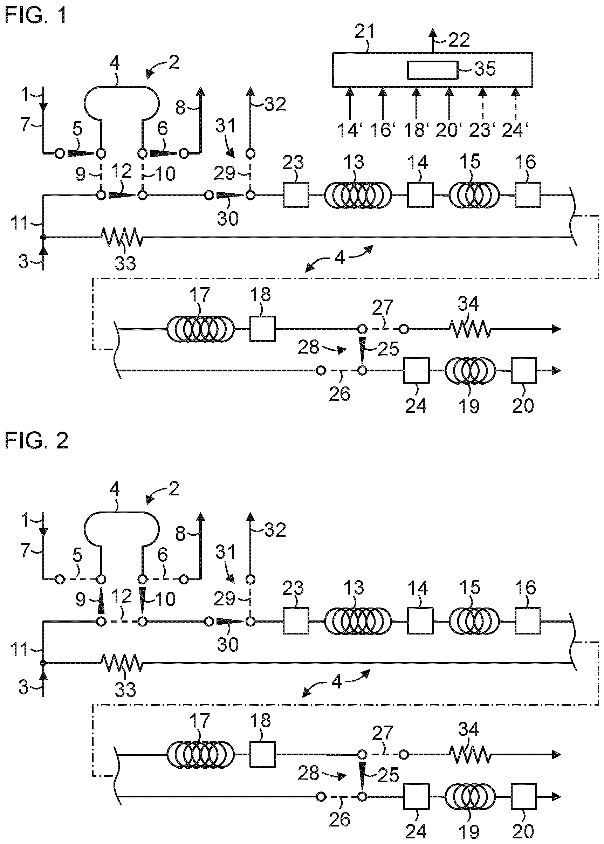

Fig. 1 bis 4 zeigen jeweils in beispielhafter schematischer Darstellung ein Blockschaltbild eines Gaschromatographen zur Analyse von Erdgas oder ähnlichen Gasgemischen 1 mit einer Dosiereinrichtung 2 zum Einschleusen einer Probe des Gasgemischs 1 in einen Trägergasstrom 3 und mit einer Trenneinrichtung 4, die zum Trennen interessierender Gaskomponenten des Gasgemischs 1 von dem Trägergasstrom 3 und der eingeschleusten Probe durchströmt wird.

Die Dosiereinrichtung 2 enthält einer Dosierschleife 4, die über ein erstes steuerbares Ventil 5 und ein zweites Ventil 6 zwischen eine Einlassleitung 7 und eine Auslassleitung 8 für das Gasgemisch 1 schaltbar ist. Die Dosierschleife 4 ist ferner über ein drittes Ventil 9 und ein viertes Ventil 10 zwischen eine Zuleitung 11 für das Trägergas 3 und die Trenneinrichtung 4 schaltbar. Ein fünftes Ventil 12 zwischen der Zuleitung 11 und der Trenneinrichtung 4 ermöglicht es, das Trägergas 3 unmittelbar in die Trenneinrichtung 4 zu leiten.

Die Trenneinrichtung 4 enthält eine erste Trennsäule 13 gefolgt von einem ersten Detektor 14, eine zweite Trennsäule 15 gefolgt von einem zweiten Detektor 16, eine dritte Trennsäule 17 gefolgt von einem dritten Detektor 18 und eine vierte Trennsäule 19 gefolgt von einem vierten Detektor 20, die in Strömungsrichtung des Trägergases 3 mit der eingeschleusten Probe des Gasgemischs 1 in einer Reihenschaltung angeordnet sind. Die Detektoren 14, 16, 18, 20 erzeugen in Abhängigkeit von ankommenden getrennten Gaskomponenten Detektorsignale 14', 16', 18', 20', die in einer an den Detektoren 14, 16, 18, 20 angeschlossenen Auswerteeinrichtung 21 (der Übersichtlichkeit halber nur in Fig. 1 dargestellt) zur quantitativen Bestimmung und Ausgabe 22 der Konzentrationen der getrennten Gaskomponenten ausgewertet werden.

Der ersten Trennsäule 13 und der vierten Trennsäule 19 kann jeweils ein zusätzlicher Detektor 23, 24 unmittelbar vorgeschaltet sein. Bei den Detektoren 14, 16, 18, 20, 23, 24 handelt es sich hier um Wärmeleitfähigkeitsdetektoren, die auf Substanzen mit unterschiedlicher thermischer Leitfähigkeit als das verwendete Trägergas ansprechen und die Gaskomponenten zerstörungsfrei detektieren. Grundsätzlich können die Gaskomponente auch auf Grundlage anderer Messprinzipien, z. B. Dichtemessung, zerstörungsfrei detektiert werden.

Zwischen dem dritten Detektor 18 und der vierten Trennsäule 19 liegt eine aus einem sechsten Ventil 25, siebten Ventil 26 und achten Ventil 27 gebildete steuerbare Umschalteinrichtung 28, die es ermöglicht, aus der dritten Trennsäule 17 eluierende Gaskomponenten entweder in die vierte Trennsäule 19 zu leiten oder an ihr vorbei aus der Trenneinrichtung 4 auszuleiten. Ferner ermöglicht es die Umschalteinrichtung 28, das aus der Zuleitung 11 kommende Trägergas 3 direkt in die vierte Trennsäule 19 und ggf. zusätzlich rückwärts in die dritte Trennsäule 17 einzuleiten. Das rückwärts in die dritte Trennsäule 17 eingeleitete Trägergas 3 kann nach Durchströmen der zweiten und ersten Trennsäule 15, 13 mittels einer aus einem neunten Ventil 29 und zehnten Ventil 30 bestehenden weiteren Umschalteinrichtung 31 über einen Ausgang 32 aus der Trenneinrichtung 4 ausgeleitet werden.

Die zehn Ventile 5, 6, 9, 10, 12, 25, 26, 27, 29, 30 sind vorzugsweise Teile einer Mehrwegeventileinheit, wie sie aus der eingangs erwähnten WO 2016/096423 A1 bekannt ist.

Fig. 1 zeigt den Gaschromatographen in einer ersten Schaltstellung der Mehrwegeventileinheit, in der die Ventile 5, 6, 12, 25, 30 geöffnet und die übrigen Ventile 9, 10, 26, 27, 29 geschlossen sind. In dieser ersten Schaltstellung wird das aus einem technischen Prozess entnommene Gasgemisch 1, z. B. Erdgas, über die Probenzuleitung 7 durch die Dosierschleife 4 geleitet und anschließend über die Auslassleitung 8 entsorgt oder in den Prozess zurückgeführt. Gleichzeitig werden die Trennsäulen 13, 15, 17, 19 und die Detektoren 14, 16, 18, 20, 23, 24 mit dem Trägergas 3, z. B. Helium oder Argon, gespült.

Fig. 2 zeigt den Gaschromatographen in einer zweiten Schaltstellung der Mehrwegeventileinheit, in der die Ventile 5, 6, 12 kurzzeitig geschlossen und gleichzeitig die Ventile 9, 10 kurzzeitig geöffnet werden, um eine vorgegebenen Probenmenge aus der Dosierschleife 4 in den Trägergasstrom 3 einzuschleusen.

Zur Beendigung der Probendosierung in den Trägergasstrom 3 werden die Ventile 5, 6, 9, 10, 12 wieder umgeschaltet, so dass die Mehrwegeventileinheit 14 wieder die in Fig. 1 gezeigte erste Schaltstellung einnimmt. Während das aus dem technischen Prozess kommende Gasgemisch 1 wieder kontinuierlich durch die Dosierschleife 4 fließt, fördert das Trägergas 3 jetzt die aus der Dosierschleife 4 entnommene Probe nacheinander durch die Trennsäulen 13, 15, 17, 19, wobei das Gasgemisch 1 in der Probe in unterschiedliche Gaskomponenten zerlegt wird, die an den Ausgängen der Trennsäulen 13, 15, 17, 19 nacheinander erscheinen und dort mit den Detektoren 14, 16, 18, 20 detektiert werden.

Die erste Trennsäule 13 ist z. B. als WCOT-Säule (wall-coated open tubular column) mit unpolarer stationärer Phase zur Trennung von n-Hexan und höheren Kohlenwasserstoffen wie Benzol, Heptan, Methylcyclohexan, Toluol und erforderlichenfalls Octan und Nonan ausgebildet, die von dem ersten Detektor 13 detektiert werden.

Die zweite Trennsäule 15 kann vom selben Typ wie die erste Trennsäule 13 aber länger als diese sein und ist zur Trennung von Butan und Pentan ausgebildet, die von dem zweiten Detektor 16 detektiert werden.

Die von n-Hexan verschiedenen Isomeren können entweder nach der ersten Trennsäule 13 in dem ersten Detektor 14 oder nach der zweiten Trennsäule 15 in dem zweiten Detektor 16 detektiert werden.

Die dritte Trennsäule 17 ist z. B. als PLOT-Säule (porous layer open tubular column) mit polarer stationärer Phase aus einem porösen Polymer zur Trennung von Kohlendioxid, Ethan Schwefelwasserstoff, Wasser und Propan ausgebildet, die von dem dritten Detektor 18 detektiert werden.

Die vierte Trennsäule 19 ist z. B. als PLOT-Säule mit Molekularsieb zur Trennung von Helium (optional), Wasserstoff, Sauerstoff, Stickstoff, Methan und erforderlichenfalls Kohlenmonoxid ausgebildet, die von dem vierten Detektor 20 detektiert werden. Für die Messung von Wasserstoff und ggf. Helium mit Wärmeleitfähigkeitsdetektoren ist die Verwendung von Argon als Trägergas 3 vorteilhaft.

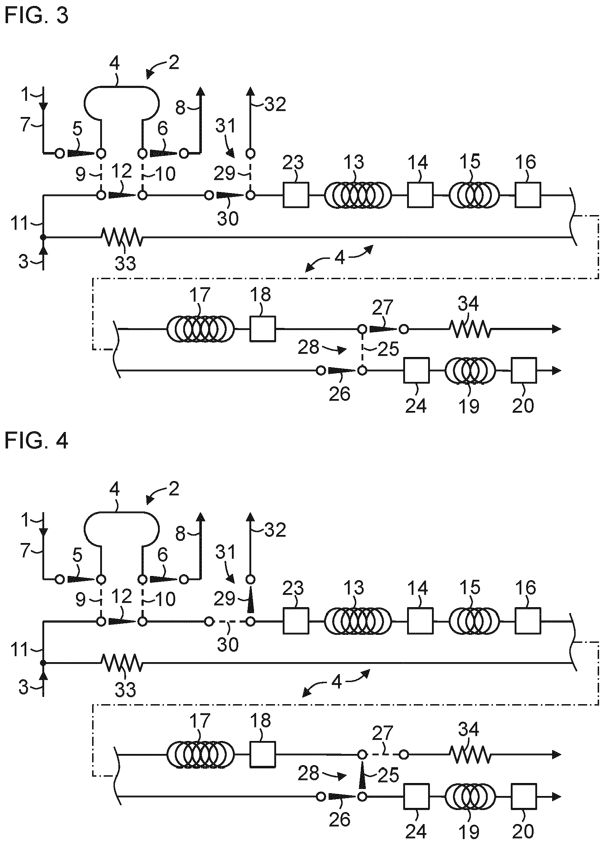

Sobald die erste von der dritten Trennsäule 17 zu trennende Gaskomponente, hier Kohlendioxid, von dem dritten Detektor 18 detektiert worden ist oder die letzte von der vierten Trennsäule 19 zu trennende Gaskomponente, hier Methan bzw. Kohlenmonoxid, von dem Detektor 24 detektiert worden ist, wird die Mehrwegeventileinheit durch Umschalten der Ventile 25, 26, 27 der Umschalteinrichtung 28 aus der in Fig. 1 gezeigten ersten Schaltstellung in in eine in Fig. 3 gezeigte dritte Schaltstellung geschaltet. In dieser dritten Schaltstellung ist das Ventil 25 geschlossen und sind die Ventile 26, 27 geöffnet.

Ein Teilstrom des Trägergases 3 gelangt jetzt über einen Strömungswiderstand 33 und durch das geöffnete Ventil 26 in die vierte Trennsäule 19, um Sauerstoff, Stickstoff, Methan und erforderlichenfalls Kohlenmonoxid weiter unterbrechungsfrei durch die vierte Trennsäule 19 zu fördern. Der andere Teilstrom des Trägergases 3 fördert die höhersiedenden Gaskomponenten weiterhin durch die erste, zweite und dritte Trennsäule 13, 15, 17 Trenneinrichtung 7 und nach ihrer Trennung und Detektion durch das geöffnete Ventil 27 und über einen weiteren Strömungswiderstand 34 aus der Trenneinrichtung 4 heraus. Auf diese Weise werden von der vierten Trennsäule (Molekularsiebsäule) 19 hochsiedende Gaskomponenten ferngehalten, die nicht auf die Molekularsiebsäule 19 gelangen dürfen, weil sie nur durch eine Konditionierung der Molekularsiebsäule 19 wieder entfernt werden können.

Die quantitative Bestimmung des Wassers und des Schwefelwasserstoffs erfolgt jeweils über einen relativen Responsefaktor des dritten Detektors 18 für Wasser bzw. Schwefelwasserstoff gegenüber einer Bezugskomponente, bei der es sich um eine der anderen von dem Detektor 18 detektierten und anschließend in der Auswerteeinrichtung 21 quantitativ bestimmten Gaskomponenten, also Kohlendioxid, Ethan und Propan handeln kann. Die beiden relativen Responsefaktoren werden vorher unter kontrollierten (Labor-) Bedingungen ermittelt und und in einem Kalibrierspeicher 35 der Auswerteeinrichtung 21 abgespeichert.

Zur Verringerung des Kalibrieraufwandes können auch die zu detektierenden höheren Kohlenwasserstoffe als n-Hexan (Benzol, Heptan, Methylcyclohexan, Toluol, Octan, Nonan) sowie die von n-Hexan verschiedenen Isomeren über relative Responsefaktoren quantitativ bestimmt werden. Im Falle der höheren Kohlenwasserstoffe als n-Hexan werden dazu im Rahmen einer Kalibration die relativen Responsefaktoren des ersten Detektors 14 für diese höheren Kohlenwasserstoffe in Bezug auf n-Hexan ermittelt und in dem Kalibrierspeicher 35 der Auswerteeinrichtung 21 abgespeichert. Wenn neben dem n-Hexan auch die übrigen Hexan-Isomeren in der ersten Trennsäule 13 getrennt und anschließend detektiert werden, werden bei der Kalibration auch relative Responsefaktoren des ersten Detektors 14 für diese Hexan-Isomeren gegenüber n-Hexan ermittelt und in dem Kalibrierspeicher 35 gespeichert. Alternativ kann dies auch für den zweiten Detektor 16 erfolgen, wenn alle Hexan-Isomeren nach Durchlaufen der zweiten Trennsäule 15 detektiert werden. Werden am Ende der zweiten Trennsäule 15 lediglich die von n-Hexan verschiedenen Isomeren detektiert, so können diese unter Verwendung von relativen Responsefaktoren des zweiten Detektors 16 für diese Hexan-Isomeren gegenüber dem von dem zweiten Detektor 16 detektierten n-Pentan quantitativ ermittelt werden.

Fig. 4 zeigt den Gaschromatographen in einer vierten Schaltstellung der Mehrwegeventileinheit, in der die Ventile 25, 26 12 geöffnet und das Ventil 27 geschlossen ist, um nach Beendigung eines Analysezyklus die Trenneinrichtung 4 mit dem Trägergas 3 zu spülen und störende Rückstände aus dem Messystem zu entfernen. Dabei werden die erste, zweite und dritte Trennsäule 13, 15, 17 rückwärts gespült und für die nachfolgende Analyse vorbereitet.

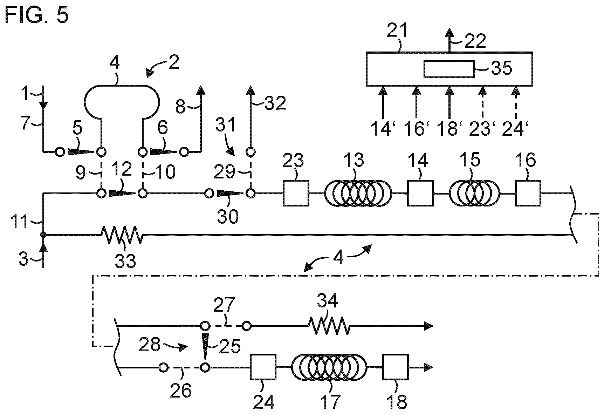

Fig. 5 zeigt ein weiteres Ausführungsbeispiel des erfindungsgemäßen Gaschromatographen, der ohne Wasserstoff, Helium, Sauerstoff, Kohlenmonoxid zu messen mit nur mit drei Trennsäulen 13, 15, 17 auskommt, wodurch die Analysenzeit verkürzt wird.

In diesem Fall ist die dritte Trennsäule 17 ebenso wie im Beispiel der Fig. 1-4 z. B. als PLOT-Säule mit polarer stationärer Phase aus einem porösen Polymer aber länger als dort zur Trennung von Stickstoff, Methan, Kohlendioxid, Ethan Schwefelwasserstoff, Wasser und Propan ausgebildet. Die steuerbare Umschalteinrichtung 28 ist zwischen dem zweiten Detektor 16 und der dritten Trennsäule 17 zur Ausschleusung von auf das Propan folgenden Eluaten aus der zweiten Trennsäule 15 angeordnet. Dadurch wird verhindert, dass Butan und weitere höhere Kohlenwasserstoffe, die in der dritten Trennsäule 17 sehr viel länger eluieren würden, auf die dritte Trennsäule 17 gelangen und dort bei nicht ausreichend langer Spülzeit in nachfolgenden Messungen als störende Komponenten auftreten würden. Die Analysenzykluszeit kann daher kurz gehalten werden.

Wenn das zu analysierende Gasgemisch 1 keinen Wasserstoff enthält, kann für das Trägergas 3 auch Wasserstoff infrage kommen.

Bei der Gesamtanalyse des Gasgemischs 1 müssen bei dem erfindungsgemäßen Gaschromatographen folgende Gaskomponenten nicht direkt kalibriert werden, sondern können mit Hilfe von relativen Responsefaktoren (RRF) bestimmt werden:
Auf dem ersten Detektor 14:
   - Isomere vom Hexan, höhere Komponenten wie Heptan, Octan, Nonan, Benzol, Toluol mit Hilfe von RRF zu n-Hexan)
Auf dem zweiten Detektor 16:
   - Neopentan (2,2-Dimethylpropan) mit Hilfe von RRF zu Isopentan oder ggf. n-Butan)
   - Optional weitere Isomere vom Hexan
Auf dem dritten Detektor 18:
   - Wasser mit Hilfe von RRF zu Ethan oder ggf. Kohlendioxid oder Propan)
   - Schwefelwasserstoff mit Hilfe von RRF zu Ethan oder ggf. Kohlendioxid oder Propan)
Auf dem vierten Detektor 20:
   - Helium mit Hilfe von RRF zu Wasserstoff)
   - Sauerstoff mit Hilfe von RRF zu Stickstoff)

Damit vereinfacht sich die benötigte Kalibrierprobe um mehr als 10 Komponenten.

## Patentansprüche

1. Gaschromatograph zur Analyse von Erdgas oder ähnlichen Gasgemischen (1)
mit einer Dosiereinrichtung (2), die eine Probe des Gasgemischs (1) in einen Trägergasstrom (3) einschleust,
mit einer von dem Trägergasstrom (3) und der eingeschleusten Probe durchströmten und Gaskomponenten des Gasgemischs (1) trennenden Trenneinrichtung (4), in der in Strömungsrichtung eine erste Trennsäule (13) gefolgt von einem ersten Detektor (14), eine zweite Trennsäule (15) gefolgt von einem zweiten Detektor (16) und eine dritte Trennsäule (17) gefolgt von einem dritten Detektor (18) in einer Reihenschaltung angeordnet sind, wobei die Detektoren (14, 16, 18) in Abhängigkeit von ankommenden getrennten Gaskomponenten Detektorsignale (14', 16', 18') erzeugen, und
mit einer an den Detektoren (14, 16, 18) angeschlossenen Auswerteeinrichtung (21), die in Abhängigkeit von den Detektorsignalen (14', 16', 18') zumindest einen Teil der getrennten Gaskomponenten quantitativ bestimmt, wobei
die erste Trennsäule (13) und der erste Detektor (14) zur Trennung bzw. Detektion von n-Hexan und höheren Kohlenwasserstoffen ausgebildet sind,
die zweite Trennsäule (15) und der zweite Detektor (16) zur Trennung bzw. Detektion der Isomeren von Butan und Pentan ausgebildet sind,
die dritte Trennsäule (17) und der dritte Detektor (18) zur Trennung bzw. Detektion von Kohlendioxid, Ethan, Schwefelwasserstoff, Wasser und Propan ausgebildet sind und wobei die Auswerteeinrichtung (21) ferner dazu ausgebildet ist, das Wasser und den Schwefelwasserstoff anhand der von dem dritten Detektor (18) für das Wasser bzw. den Schwefelwasserstoff gelieferten Detektorsignale (18') und in einem Kalibrierspeicher (35) der Auswerteeinrichtung (21) gespeicherten relativen Responsefaktoren des dritten Detektors (18) für Wasser und Schwefelwasserstoff zu jeweils einer Bezugskomponente, insbesondere zu Ethan, Kohlendioxid oder Propan, quantitativ zu bestimmen.

2. Gaschromatograph nach Anspruch 1, **dadurch gekennzeichnet, dass** die Auswerteeinrichtung (21) dazu ausgebildet ist, die höheren Kohlenwasserstoffe als n-Hexan anhand der von dem ersten Detektor (14) für sie gelieferten Detektorsignale (14') und von in dem Kalibrierspeicher (35) der Auswerteeinrichtung (21) gespeicherten relativen Responsefaktoren des ersten Detektors (14) für diese höheren Kohlenwasserstoffe zu n-Hexan quantitativ zu bestimmen.

3. Gaschromatograph nach Anspruch 1 oder 2, **dadurch gekennzeichnet, dass** die erste Trennsäule (13) und der erste Detektor (14) weiterhin zur Trennung bzw. Detektion der von n-Hexan verschiedenen Hexan-Isomeren ausgebildet sind und dass die Auswerteeinrichtung (21) ferner dazu ausgebildet ist, diese Hexan-Isomeren anhand der von dem ersten Detektor (14) für sie gelieferten Detektorsignale (14') und von in dem Kalibrierspeicher (35) der Auswerteeinrichtung (21) gespeicherten relativen Responsefaktoren des ersten Detektors (14) für diese Hexan-Isomeren zu n-Hexan quantitativ zu bestimmen.

4. Gaschromatograph nach Anspruch 1, 2 oder 3, **dadurch gekennzeichnet, dass** die zweite Trennsäule (15) und der zweite Detektor (16) weiterhin zur Trennung bzw. Detektion der Hexan-Isomeren ausgebildet sind und dass die Auswerteeinrichtung (21) ferner dazu ausgebildet ist, die von n-Hexan verschiedenen Hexan-Isomeren anhand der von dem zweiten Detektor (16) für sie gelieferten Detektorsignale (16') und von in dem Kalibrierspeicher (35) der Auswerteeinrichtung (21) gespeicherten relativen Responsefaktoren des zweiten Detektors (16) für diese Hexan-Isomeren zu n-Hexan quantitativ zu bestimmen.

5. Gaschromatograph nach Anspruch 1, 2 oder 3, **dadurch gekennzeichnet, dass** die zweite Trennsäule (15) und der zweite Detektor (16) weiterhin zur Trennung bzw. Detektion der von n-Hexan verschiedenen Hexan-Isomeren ausgebildet sind und dass die Auswerteeinrichtung (21) ferner dazu ausgebildet ist, diese Hexan-Isomeren anhand der von dem zweiten Detektor (16) für sie gelieferten Detektorsignale (16') und von in dem Kalibrierspeicher (35) der Auswerteeinrichtung (21) gespeicherten relativen Responsefaktoren des zweiten Detektors (16) für diese Hexan-Isomeren zu n-Pentan quantitativ zu bestimmen.

6. Gaschromatograph nach einem der vorangehenden Ansprüche, **dadurch gekennzeichnet, dass** die dritte Trennsäule (17) und der dritte Detektor (18) weiterhin zur Trennung bzw. Detektion von Stickstoff und Methan, ggf. auch Wasserstoff, ausgebildet sind und dass zwischen dem zweiten Detektor (16) und der dritten Trennsäule (17) eine steuerbare Umschalteinrichtung (28) zur Ausschleusung von auf das Propan folgenden Eluaten aus der zweiten Trennsäule (15) vorgesehen ist.

7. Gaschromatograph nach einem der Ansprüche 1 bis 5, **dadurch gekennzeichnet, dass** eine vierte Trennsäule (19) und ein vierter Detektor (20) zur Trennung bzw. Detektion von Wasserstoff, Sauerstoff, Stickstoff, Methan ggf. auch Helium und Kohlenmonoxid ausgebildet sind und dass zwischen dem dritten Detektor (18) und der vierten Trennsäule (19) eine steuerbare Umschalteinrichtung (28) zur Ausschleusung von dem aus der dritten Trennsäule (17) eluierenden Kohlendioxid und folgenden Eluaten vorgesehen ist.
